# EUROPEAN PATENT SPECIFICATION

(11) **EP 4 366 803 B1**
(45) Date of publication and mention of the grant of the patent: **06.08.2025**
(21) Application number: 22740804.4
(22) Date of filing: 27.06.2022
(51) Int. Cl.: A61M 15/00, A61B 5/087, G09B 23/28

(54) **DEVICES AND A SYSTEM FOR DETECTION AND ANALYSIS OF INHALER USE**
VORRICHTUNGEN UND SYSTEM ZUR ERKENNUNG UND ANALYSE DER INHALATORVERWENDUNG
DISPOSITIFS ET SYSTÈME DE DÉTECTION ET D'ANALYSE D'UTILISATION D'INHALATEUR

(30) Priority: 08.07.2021 US 202163219401 P
(43) Date of publication of application: 15.05.2024
(73) Proprietor: Astrazeneca AB, 151 85 Södertälje (SE)
(72) Inventor: SELBY, Robert, Melbourn Royston Hertfordshire SG8 6EE (GB); PETRUS, Andrei, Melbourn Royston Hertfordshire SG8 6EE (GB); KOHUT, Pavel, Melbourn Royston Hertfordshire SG8 6EE (GB); HORNE, David, Melbourn Royston Hertfordshire SG8 6EE (GB); SAVOV, Svilen, Melbourn Royston Hertfordshire SG8 6EE (GB)
(74) Representative: AstraZeneca Intellectual Property
(86) International application number: PCT/EP2022/067611
(87) International publication number: WO 2023/280622

(56) References cited:
- WO-A1-2006/110080
- WO-A1-2014/033229
- WO-A1-2015/144442
- WO-A1-2016/081294
- WO-A1-2020/222146
- WO-A1-2021/116971
- WO-A1-95/26769
- WO-A2-2015/006701
- US-A- 5 333 106
- US-A1- 2006 254 581
- US-A1- 2012 055 472

## Description

### Field of Invention

The present application relates to devices, methods and a system for detection and analysis of inhaler use, in particular breath detection modules and inhaler device counters.

### Background

Many types of medicines are provided in fluid form, such as a solution or suspension of particles in a propellant or emulsion, and are adapted for oral inhalation by a patient. As one example, a container might contain asthma medicine such as fluticasone propionate.

In order to deliver medicine to the patient, the container operates in conjunction with an actuator as a system commonly known as a pressurised metered dose inhaler (pMDI) system. The actuator includes a housing having an open container-loading end and an open mouthpiece. A nozzle element is disposed within the housing and includes a valve stem-receiving bore communicating with a nozzle orifice. The orifice is aimed toward the mouthpiece. In order to receive a properly metered dosage of medicine from the container, the patient installs the container into the actuator through the container-loading end until the valve stem is fitted into the receiving bore of the nozzle element. With the container so installed, the opposite end of the container typically extends to some degree outside the actuator housing. The patient then places the mouthpiece into his or her mouth and pushes downwardly on the exposed container end. This action causes the container to displace downwardly with respect to the valve stem, which in turn unseats the valve. Owing to the design of the valve, the design of the nozzle element, and between the interior of the container and the ambient air, a short burst of precisely metered, atomized medicine is thereby delivered to the patient.

Such a container is filled with a predetermined volume of active substance, i.e. medicine. Hence, the container can nominally deliver a predetermined number of medicine doses before it has to be discarded. In order to visualize the number of remaining doses in such an inhaler device, it is preferably provided with a counter that displays the amount of medicine remaining in the container. Thus, the counter gives an indication of when to replace the inhaler device or container. The display of the "present state" can either be done in absolute terms, e.g. by showing in figures the actual number of doses that are still available, or in relative terms, e.g. by a color gradient from one color to another.

The Food and Drug Administration (FDA), (2003) Guidance for Industry: Integration of Dose-Counting Mechanisms into MDI Drug Products distinguishes between overcounting (where a count is recorded although no dose has been fired) and undercounting (where a dose has been fired but not counted). Undercounting is the more dangerous failure mode because it can ultimately lead to the user thinking they still have puffs available in the canister when it becomes empty. The FDA recommends that whilst overcounting is undesirable, undercounting should be avoided if possible.

WO 2006/110080 discloses a mechanical inhaler counter comprising a counter housing, a rocker arm with a pawl, the rocker arm being pivotally supported by the housing and arranged to perform a rocker movement in response to a linear actuation motion, a return spring for resetting the rocker arm, a rachet wheel engageable with the pawl to convert the movement of the rocker arm into an incremental rotational motion of an axle arrangement advancing a display means, the axle arrangement further comprising a back rotation prevention means in the form of a spring loaded friction brake and worm gear, and the display means comprising a rotatable indicator means with teeth that engage the worm-gear. WO2021116971A1 discloses an electronic inhaler counter comprising a rocker arm 700, fig.33 which can pivot to engage a count switch 612. There is no return spring coupled to a rocker arm pivot.

### Summary of the Invention

Aspects of the invention are as set out in the independent claim and optional features are set out in the dependent claims. Aspects of the invention may be provided in conjunction with each other and features of one aspect may be applied to other aspects.

Aspects of the disclosure are related to counting doses dispensed by an inhaler. The electronic counter design implements a displacement-based counting principle. This approach is inherently more reliable than force-based counting as significant force variation is often seen through container use life, ambient temperature and humidity, and the orientation of the applied force. In use, the electronic inhaler counter may register a count early in the container stroke, this may reduce the chance of undercounting the dispensed doses. A possible advantage of the operating principle employed in this design is that the switching point is well defined as the sprung pivot may remain in position until after the switch is actuated, this may also reduce the chance of undercounting the dispensed doses. It may also make good use of the available space and the geometry can be tuned for best operation. A digital display may also allow the displayed figures to be larger relative to small sized figures on mechanical inhaler counters required to fit in the inhaler device, thus the use of a digital display may enhance the readability of the display.

Aspects of the disclosure also relate to detecting and analysing breath technique during use of the inhaler. Inspiration rate is important when a patient uses an inhaler as it can affect the transport of drug. For pMDls, lower flow rates are generally seen as beneficial in literature as they promote better deposition of drug into the lung.

The breath detection solution may be advantageous as it can alert a user if a dose was improperly administered which may affect the efficacy of treatment. Furthermore, the breath detection solution may enable a user to receive feedback to improve their inhaler breath technique which may improve the efficacy of future treatment and, thus, improve management of the user's condition. Good coordination of user inhalation with delivery of the dose is critical to the efficacy of treatment and is, therefore, important for effective management of the user's condition. Potential modes of misuse may include, but are not limited to, incorrect timing of delivery/breath, not inhaling, sub-optimal inhalation flow profile (i.e. too fast or too slow), and no breath-hold after inhaling.

The breath detection solution may be advantageous by accurately detecting breath at flow rates as low as 30 I/min which, from literature, is the lowest flow rate achieved in practice by users trained to inhale correctly through a pMDI.

The breath detection solution may be advantageous by not impacting the existing air flow path through an inhaler device. This may allow compatibility with existing inhalers, whilst not affecting or interfering with the dispensing and delivery of the drug. The breath detection solution may also be advantageous as the electronics and sensors for breath detection may fit within the existing mechanical inhaler adapter counter module (ACM) footprint, such as that disclosed in WO 2006/110080. This may allow it to be compatible with existing inhalers, and inhaler counter systems.

The breath detection solution may also be advantageous as the electronics and sensors for breath detection may be low in cost at high volumes as for hygienic reasons it is preferred that the entire inhaler device is disposable.

In a first aspect there is provided an electronic inhaler counter for counting linear actuation of a pressurised metered dose inhaler, pMDI, the electronic inhaler counter comprising:
a rocker arm comprising a proximal end providing a pivot and a distal end providing a head;
a return spring coupled to the rocker arm pivot; and
a count switch;
wherein, in response to a first selected degree of linear actuation motion, the rocker arm is arranged to perform a first rocker movement and engage the count switch with the rocker head; and in response to further linear actuation motion, the spring is engaged to enable the rocker arm to perform a second rocker movement, such that the rocker head maintains engagement with the count switch.

In some examples, the first selected degree of linear actuation motion engages a first portion of the rocker arm with the actuator tongue to rock the rocker arm in a first direction to engage the rocker head with the count switch, and wherein further linear actuation motion engages a second portion of the rocker arm to rock the rocker arm in a second direction to maintain engagement of the rocker head with the count switch.

In some examples, the first rocker movement is an anti-clockwise rotation about the pivot, and the second rocker movement is a clockwise rotation about the count switch.

In some examples, the first selected degree of linear actuation motion does not compress the return spring, and wherein further linear actuation motion compresses the return spring. In some examples, this may allow the sprung pivot to remain in position until after the switch is actuated, thus providing a well-defined switching point.

In some examples, the electronic inhaler counter further comprises a spring retainer, wherein the spring retainer houses the end of the spring not coupled to the pivot. This may allow a longer spring to be used in the device than would otherwise be possible, hence allowing further overtravel.

In some examples, the electronic inhaler counter is configured to attach to the end of an inhaler container mounted in an inhaler actuator housing, wherein the linear actuation motion is relative to the actuator housing and the rocker arm is configured to engage with an actuator tongue coupled to the actuator housing.

In some examples, the electronic inhaler counter is configured to fit within at least a portion of the inhaler actuator housing. In some examples, the inhaler device is actuated by depressing the electronic inhaler counter with respect to the actuator housing. In some examples, the inhaler device is actuated at least a portion of the electronic inhaler counter fits within the inhaler actuator housing.

In some examples, the electronic inhaler counter further comprises a digital display. The display may be configured to display the absolute number of linear actuations/dispensed doses of the inhaler device counted by the electronic inhaler counter. The display may be configured to display the absolute number of linear actuations/doses of the inhaler device remaining. The display may be further configured to display a relative indication of the number of linear actuations/doses of the inhaler device remaining. In some examples, the display may provide both a relative and exact indication of the remaining number of doses of an inhaler device. The number of linear actuations/doses of the inhaler device remaining may be calculated by the counted absolute number of linear actuations of the inhaler device subtracted from a predetermined maximum number of linear actuations/doses of the inhaler device. A digital display may allow the displayed figures to be larger relative to small sized figures on mechanical inhaler counters to fit in the inhaler device, this may enhance the readability of the display.

In some examples, the electronic inhaler may be further configured to record time stamp data of linear actuation of the inhaler. In some examples, the time stamp data of linear actuation of the inhaler may comprise time stamp data of the dose count. In some examples, the time stamp data of linear actuation of the inhaler may comprise time stamp data of the fire point. In some examples, the time stamp data of linear actuation of the inhaler may comprise both time stamp data of the dose count and the fire point.

The dose count may be different to the fire point of the inhaler as the fire point is triggered by the amount of compression of the inhaler container with respect to the actuator body that is necessary for delivering a dose of medicament; whereas the dose count is triggered by the amount of compression of the inhaler container with respect to the actuator body that is necessary for affecting the electronic inhaler counter to count one dose i.e. to cause the rocker arm to engage the count switch. Since undercounting is undesirable due to the risk that the user believes that there is medicament left in the inhaler container when it actually is empty, the dose count point may be set to be a predetermined amount less than the fire point, whereby firing without counting is effectively avoided.

In another aspect there is provided an inhaler breath detection module for detecting the beginning and/or end of an inhalation breath;
the breath detection module being coupled to an airpath of the inhaler;
wherein the breath detection module comprises a sensing means configured to provide signals indicative of a change in parameter in the inhaler airpath as a function of time caused by an inhalation breath;
wherein the breath detection module comprises a controller configured to determine the presence of breath, based on a change in parameter in the inhaler airpath as a function of time.

In some examples, the controller may be further configured to determine the duration of breath, based on a change in parameter in the inhaler airpath as a function of time. In some examples, the controller is further configured to determine either: (i) the confidence level of the beginning and/or end of a breath; or (ii) estimated flow rate; based on a change in parameter in the inhaler airpath as a function of time. In some examples, the controller is further configured to determine both (i) the confidence level of the beginning and/or end of a breath; and (ii) estimated flow rate; based on a change in parameter in the inhaler airpath as a function of time.

In some examples, the controller may be further configured to record time stamp data of a breath. In some examples, the time stamp data of a breath may comprise time stamp data of the start of a breath. In some examples, the time stamp of a breath may comprise time stamp data of the end of a breath. In some examples, time stamp data of a breath may comprise time stamp data of the start and end of a breath.

In some examples, at least a portion of the inhaler breath detection module is configured to fit within at least a portion of the inhaler actuator housing. In some examples, the inhaler device is actuated by depressing the breath detection module with respect to the actuator housing. In some examples, when the inhaler device is actuated at least a portion of the breath detection module fits within the inhaler actuator housing. In some examples, the inhaler breath detection module is configured to attach to the end of an inhaler container mounted in an inhaler actuator housing.

In some examples, the sensing means is configured to detect a change in parameter of an airflow through a portion of the actuator housing between the actuator housing and the container.

In some examples, the sensing means may comprise a pressure sensor. In some examples, at least one differential pressure sensor may be used with one port connected to an appropriate location inside the device and with the other port open to atmosphere. In some examples, at least one absolute pressure sensor may be used. In some examples, the at least one absolute pressure sensor may be a miniature board mount barometric pressure sensor. In some examples, two absolute pressure sensors may be used where one measures atmospheric pressure and the other pressure inside the device. In some examples, a single absolute sensor may be used that measures pressure inside the device and tracks changes in pressure over time. One advantage of using pressure sensors may be that pressure drop readings provide a more direct measure of flow rate, and they are not susceptible to sound noise. One advantage of using at least one absolute pressure sensor may be that they are cheap and small to address form factor and cost constraints. One advantage of using a differential pressure sensor may be they are more accurate than absolute pressure sensors.

In some examples, the sensing means may comprise a microphone. One advantage of using a microphone as a sensor may be that they are cheap and small to address form factor and cost constraints. Another advantage of using a microphone may be that they do not require a reliably sealed port. Another advantage of using a microphone may be that it is more accurate at detecting low flow rates than a pressure sensor of equivalent cost and size.

In some examples, the inhaler breath detection module may further comprise an acoustic feature. In some examples, the acoustic feature may be configured to alter a property of the airflow sensed by the sensing means. In some examples, the acoustic feature may comprise a narrowing or restriction through which a portion of the airflow may be configured to flow when a user takes a breath. In some examples, the acoustic feature may comprise a narrowing or restriction of the distance between the inhaler breath detection module and the inhaler actuator housing.

In some examples, the sensing means is arranged to be adjacent to the acoustic feature. In some examples wherein the sensing means comprises a microphone, the microphone may be configured to detect sound generated as air flows through the acoustic feature. In some examples, the acoustic feature may comprise an orifice, coupled to the airpath, configured to generate "jet noise" or whistling. In some examples, the acoustic feature may be used to amplify the signal recorded by the microphone, this may increase the signal to noise ratio.

In some examples, the inhaler breath detection module may comprise a plurality of sensing means each configured to provide signals indicative of a change in parameter. In some examples, the plurality of sensing means comprises at least two microphones. In some examples, the controller may be configured to perform noise cancellation based on a comparison of the signals indicative of a change in parameter from the plurality of sensing means. This may provide improved breath detection accuracy in noisy environments.

In some examples, the controller is programmed with a machine learning algorithm, wherein the algorithm is trained using training parameters. In some examples the training parameters relate power at each frequency to different weightings. An advantage of using a machine learning approach may be to improve the association between the microphone sensing signal and flow rate, particularly in different noise environments. This may improve the accuracy of flow rate and breath determination in different noise environments.

In some examples, the controller is configured to process the signals indicative of the change in parameter into the frequency domain. In some examples, the controller is configured to apply a weighting to the power at each frequency. The weightings may be pre-programmed and are determined by training a machine learning algorithm across a wide data set including inhalation samples and noise samples across a range of flow rates.

In some examples, the controller is configured to determine the cepstrum of the signals indicative of the change in parameter. In some examples, the cepstrum may contain information about the rate of change in different spectrum bands. In some examples the cepstrum is useful in picking out the base frequencies and harmonics.

In some examples, the controller is configured to perform linear regression, for example to determine an estimate of the flow rate. In some examples, the controller is configured to perform logistic regression, for example to output a confidence level of the presence of breath, based on stored training parameters. In some examples, the confidence level of the presence of breath may be determined for the start of a breath, and/or the end of a breath. In some examples, the controller is configured to perform both linear regression, for example to determine a flow rate estimate, and logistic regression, for example to output a confidence level of the presence of breath.

In some examples, the inhaler breath detection module comprises a communications interface. In some examples, the inhaler breath detection module comprises a short-range wireless communications interface. In some examples, the controller may be configured to send data comprising either: (i) the confidence level of the beginning and/or end of a breath, or (ii) the estimated flow rate to a remote device, via the short-range wireless communications interface. In some examples, the controller may be configured to send data comprising both: (i) the confidence level of the beginning and/or end of a breath, and (ii) the estimated flow rate to a remote device, via the short-range wireless communications interface. In some examples, the short-range wireless communications interface may use Bluetooth^{®} communication. In other examples, other short-range wireless communication interfaces may be used, including but not limited to WiFi, near-field communication (NFC), ZigBee, and radiofrequency identification (RFID).

In some examples, the inhaler breath detection module may further comprise an accelerometer. In some examples, the accelerometer may be configured to detect the orientation of the device. In some examples, the accelerometer may be configured to detect shaking of the device. In some examples, the accelerometer may be configured to detect both orientation of the device and shaking of the device. In some examples, the inhaler breath detection module may be configured to record shake data prior to a puff, for example 5 seconds prior to a puff. Recording shaking of the device may be advantageous for providing data on the priming of the device prior to an actuation event. For example, in some examples the inhaler breath detection module may be configured to detect the presence of priming/shaking prior to an actuation event. In some examples, the inhaler breath detection module may be configured to record the duration or intensity of priming/shaking.

In another aspect there is provided an inhaler breath detection and analysis system, comprising an inhaler breath detection module and an electronic inhaler counter.

In some examples, the inhaler breath detection and analysis system further comprises a housing, wherein both the inhaler breath detection module and the electronic inhaler counter are disposed within the housing.

In some examples, the inhaler breath detection and analysis system may be configured to fit within the footprint of an existing mechanical inhaler counter, such as that disclosed in WO 2006/110080.

In some examples, the inhaler breath detection and analysis system is configured to attach to an inhaler container.

In another aspect there is provided an inhaler technique feedback app configured to:
receive a signal from an inhaler breath detection module, wherein the signal comprises data including at least one of a confidence level of breath, and/or an estimate flow rate of breath;
perform post-processing of the data; and
provide breath technique feedback, based on the at least one of the confidence level of breath and/or the flow rate of breath relative to pre-determined optimal ranges of use.

In some examples, the app is configured calculate the breath duration using the confidence level of breath. For example, the app is configured to calculate the breath duration using the confidence level of the start and end of a breath.

In some examples, the signal received by the inhaler technique feedback app from the inhaler breath detection module further comprises breath duration, time stamp data of breath, and/or shake data.

In some examples, postprocessing procedures may include procedures to correct noisy, imprecise, or non-user-friendly knowledge derived by the algorithm. In some examples, the post-processing of data includes filtering, for example using Gaussian filters or morphological filters. In some examples, filtering can be used to smooth out the data, and/or to remove outlier "blips". In some examples post-processing may also include various pruning routines, rule quality processing, rule filtering, rule combination, model combination, or knowledge integration.

In some examples, the inhaler technique feedback app may be further configured to receive a signal from an electronic inhaler counter, wherein the signal comprises data. In some examples, the data may include time stamp data of at least one linear actuation of the inhaler. In some examples, the time stamp data of the at least one linear actuation of the inhaler may comprise the time stamp data of the dose count. In some examples, the time stamp data of the at least one linear actuation of the inhaler may comprise the time stamp data of the fire point. In some examples, the time stamp data of the at least one linear actuation of the inhaler may comprise both time stamp data of the dose count and the fire point.

In some examples, the inhaler technique feedback app may be further configured to provide an indication to the user of how many doses have been administered in a certain time period, for example, in a day. In some examples, the inhaler technique feedback app may be further configured to provide an indication to the user about what time at least one dose was administered. This may help the user track their dose history, important for effective management of the user's treatment and/or condition.

In some examples, the inhaler technique feedback app may be further configured to:
receive the signal from the inhaler breath detection module, wherein the signal comprises data including time stamp data of at least one breath;
receive a signal from an electronic inhaler counter, wherein the signal comprises data including time stamp data of at least one linear actuation of the inhaler;
compare the time stamp data of the at least one breath relative to the time stamp data of the at least one linear actuation of the inhaler; and
provide breath technique feedback to the user, based on the timing of breath relative to the timing of inhaler actuation.

In some examples, the inhaler technique feedback app may be further configured to provide an indication to the user if the timing of the breath was outside a pre-determined time range relative to the timing of inhaler actuation, for example, if breath was too early or too late relative to inhaler actuation.

In some examples, the inhaler technique feedback app may be configured to provide technique feedback based on aggregated data and/or long-term use patterns. This may help the user address potential reoccuring modes of inhaler misuse, for example, incorrect timing of delivery/breath, not inhaling, sub-optimal inhalation flow profile (i.e. too fast or too slow), and/or no breath-hold after inhaling.

In some examples, the app may be configured to detect if a breath is longer than a predetermined time period, for example 2 s. In some examples, the app may be configured to detect is a breath has been taken after firing. In some examples, the app may be configured to detect if breath flow rate is maintained below a predetermined rate, for example 100 I/min. In some examples, the app may be able to detect if no breath is associated with an actuation. In some examples, the app may be able to detect if no breath-hold is associated with an actuation.

In some examples, the app may be configured to receive shake data from the inhaler breath detection module. In some examples, the app may be able to detect if no priming/shaking of the inhaler is associated prior to an actuation. In some examples, the app may be configured to detect if the duration and/or intensity of shaking was outside a pre-determined range.

In another aspect there is provided an inhaler system comprising: an inhaler actuation housing; an inhaler container; and an electronic inhaler counter.

In another aspect there is provided an inhaler system comprising: an inhaler actuation housing; an inhaler container; and an inhaler breath detection module.

In some examples, the inhaler system comprises an inhaler actuation housing; an inhaler container; an electronic inhaler counter; and an inhaler breath detection module.

In some examples, the inhaler breath detection module and/or electronic inhaler counter are attached to the inhaler container. In some examples, the inhaler breath detection module and electronic inhaler counter are disposed within an breath detection and analysis system housing, wherein the breath detection and analysis system housing is configured to attach to the inhaler container.

In some examples, the inhaler breath detection module and/or electronic inhaler counter are configured to fit within the footprint of an existing mechanical inhaler counter, for example the footprint of that disclosed in WO 2006/110080. In some examples, the breath detection and analysis system housing is configured to fit within the footprint of an existing mechanical inhaler counter, for example the footprint of that disclosed in WO 2006/110080.

### Brief Description of Drawings

Embodiments of the disclosure will now be described, by way of example only, with reference to the accompanying drawings, in which:
Figure 1. shows a cross-section of an example electronic inhaler counter.
Figure 2. shows a schematic flow diagram of a method of use of an electronic inhaler counter.
Figures 3A to C. shows an example cross-section of an electronic inhaler counter in use.
Figure 4. shows a perspective view of an example inhaler assembly comprising an electronic inhaler counter.
Figure 5. shows a schematic illustration of an inhaler breath detection module.
Figures 6A and B. shows example section-views of an inhaler breath detection module, mounted in a housing.
Figure 7. shows an example section-view of an acoustic feature and microphone position in an inhaler breath detection module, mounted in a housing.
Figure 8. shows a schematic flow diagram of a method of signal processing by an inhaler breath detection module.
Figures 9 and 9B. show schematic flow diagram of a method of signal processing and analytics by an inhaler technique feedback app.

### Detailed Description

### Electronic inhaler counter

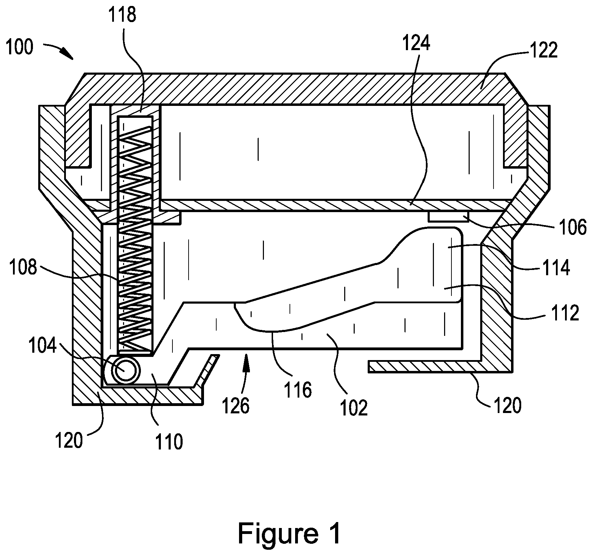

Figure 1 shows an example electronic inhaler counter 100 for counting linear actuation of a pressurised metered dose inhaler, pMDI. The electronic inhaler counter 100 comprises a housing 120. The housing 120 is the arranged such that the longitudinal axis is parallel to the direction of linear actuation.

Within the housing 120, the electronic inhaler counter 100 of Figure 1 comprises a rocker arm 102, the rocker arm 102 comprising a proximal end 110 providing a pivot 104 and a distal end 112 providing a head 114. The rocker arm 102 comprises a portion of increased thickness further comprising a rigid rocker section 116 located between the proximal 110 and distal 112 ends of the rocker arm 102. In the example shown, the rocker section 116 has a dogleg shape comprising a curved central portion. In other examples, the rocker section 116 can have other shapes or geometries comprising a curved central portion to provide a central pivot. The portion of increased thickness of the rocker arm 102 including the rocker section 116 is configured to increase the thickness of the rocker arm 102 parallel to the transverse direction, such that the rocker section 116 protrudes from the main body of the rocker arm 102. The head 114 extends from the distal end 112 of the rocker arm 102 roughly parallel to the longitudinal axis of device 100. In this example, the head 114 has a round fillet shape. In other examples, the head 114 may be a different shape, for example a convex dome, or a flat surface. The head 114 preferably has a large surface area and may be disposed on the portion of the rocker arm 102 of increased thickness. The head 114 is sized to contact a count switch 106.

The electronic inhaler counter 100 further comprises the count switch 106. The count switch 106 is arranged above the rocker arm head 114. The count switch 106 is coupled to the underside of a printed circuit board assembly (PCBA) 124. The PCBA is arranged parallel to the transverse axis of the counter device 100 and is disposed above the rocker arm 102, within the housing 120. The PCBA 124 may be sized to fit the interior footprint of the housing 120.

The electronic inhaler counter 100 further comprises a pre-compressed return spring 108, arranged parallel to the longitudinal axis of the counter device 100 such that the spring 108 axis of compression is parallel to the direction of linear actuation. One end of the spring 108 is coupled to the rocker arm pivot 104. In this example, the other end of the spring 108 is retained by a spring retainer cap 118, wherein a portion of the spring 108 fits within the cavity of the spring retainer cap. The spring retainer cap 118 is arranged to be parallel to the longitudinal axis of the counter device 100. The spring retainer cap may extend beyond the PCBA 124 and may be retained by a push fit within the housing 120. An advantage of the retainer cap 118 may be to allow a longer spring to be used in the counter device 100 than would otherwise be possible, hence allowing further overtravel and the ability to provide the required force without reaching solid height. In other examples not comprising a spring retainer cap 118, the end of the spring 108 not coupled to the pivot 104 may be coupled to the underside of the PCBA 124, or otherwise directly coupled to the housing 120.

In the preferred example, the PCBA 124 is powered by a battery (not shown). In some examples, the battery is a coin cell battery. In some examples, the battery is coupled to the upper surface of the PCBA 124, on the opposite surface to the count button 106.

In the preferred example, the PCBA 124 is further coupled to a display (not shown). In this example, the display is a digital display. In some examples, the digital display may be an LCD screen. The digital display is arranged on the top surface of the counter device 100. In other examples, the display screen may be located at other positions on the housing, for example, the side. In this example, the display is secured using a bezel 122, coupled to the housing 120.

In the preferred example, the housing 120 has an opening 126 on the bottom surface of the counter device 100 opposite the digital display, underneath the rocker arm 102. The opening 126 is arranged parallel to the transverse axis of the counter device 100. The opening 126 is arranged to expose at least the rocker section 116 of the rocker arm 102.

The electronic inhaler counter 100 is arranged wherein, in response to a first selected degree of linear actuation motion, the pre-compressed spring 108 holds down the pivot 104 and the rocker arm 102 is configured to perform a first rocker movement until the count switch 106 is engaged with the rocker head 114. In the example shown, the first rocker movement is an anti-clockwise rotation.

In response to further linear actuation motion, the spring 108 is configured to engage which displaces the pivot 104 and enables the rocker arm 102 to perform a second rocker movement. In the example shown, the spring 108 is compressed and vertically displaces the pivot 104. This causes the rocker section 116 of the rocker arm 102 to pivot the rocker arm 102 in a clockwise direction.

In response to the removal of biasing force, the spring 108 is configured to return to its original configuration, pushing down the pivot 104 and returning the rocker arm 102 to its original configuration.

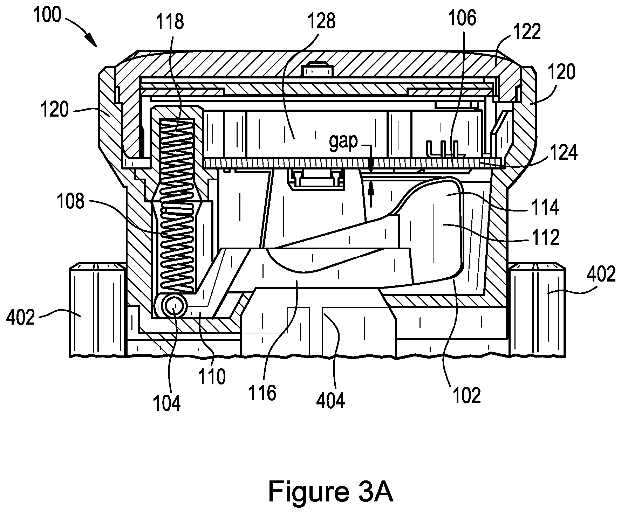

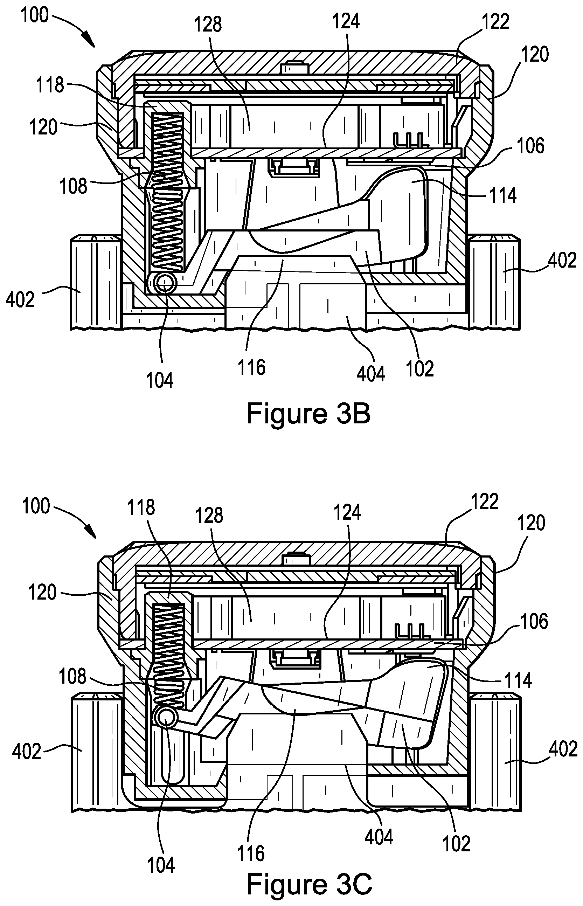

In some examples, the electronic inhaler counter 100 is configured to attach to the end of an inhaler container mounted in an inhaler actuator housing, for example as shown in more detail in Figure 3 below, wherein the linear actuation motion is relative to the actuator housing and the rocker arm 102 is configured to engage with an actuator tongue coupled to the actuator housing. In some examples, the portion of increased thickness of the rocker arm 102 comprising the rocker section 116 is configured to engage with the actuator tongue.

In this example, the rocker head 114 maintains engagement with the count switch 106 during the second rocker movement.

In this example, the first rocker movement is an anti-clockwise rotation at the pivot 104. In this example, the second rocker movement is a clockwise rotation of the rocker arm 102 about the count switch 106, enabled by compression of the spring 108.

The opening 126 in the housing 120 may be configured to receive an actuator tongue of an inhaler device. In some examples, linear actuation of the inhaler counter causes the opening 126 to advance onto the actuator tongue of an inhaler device. In response to a first selected degree of linear actuation motion, at least the rocker section 116 of the rocker arm 102 contacts the actuator tongue. In response to further linear actuation motion, the at least rocker section 116 of the rocker arm 102 maintains contact with the actuator tongue.

In the example shown in Figure 1, in response to a first selected degree of linear actuation motion, the rocker section 116 of the rocker arm 102 is configured to contact the actuator tongue of the inhaler device which causes the first rocker movement.

In the example shown in Figure 1, in response to further linear actuation motion, the rocker section 116 of the rocker arm 102 is configured to pivot at the contact point with the actuator tongue of the inhaler device during the second rocker movement.

The electronic inhaler counter 100 must reliably detect when a dose has been dispensed from the container. It should do this in such a way that there is no significant addition to the force a user must supply to depress the container, for example < 5 N added. The mechanism must also allow significant over travel, such that the switching mechanism does not stop the device from fully actuating the container.

The force required to register a count is driven mainly by the force required to activate the switch 106 and the mechanical advantage of the rocker arm 102. The key requirement is that the lever arm pivot 104 is maintained during the first rocker movement until contact with the count switch 106 is made.

In the example wherein the first rocker movement is actuated by contact between the rocking section 116 or the rocker arm 102 and an actuator tongue on the inhaler actuator body, the pivot point 104 may be held down. This may be advantageous by providing a defined switching point. However, the device 100 may also allow significant over travel, hence the pivot 104 is held down by the pre-compressed spring 108. The spring 108 can be further compressed to allow significant over travel but may be sufficiently stiff that the pivot 104 does not move until the count button 106 has registered a count in response to a first selected degree of linear actuation motion.

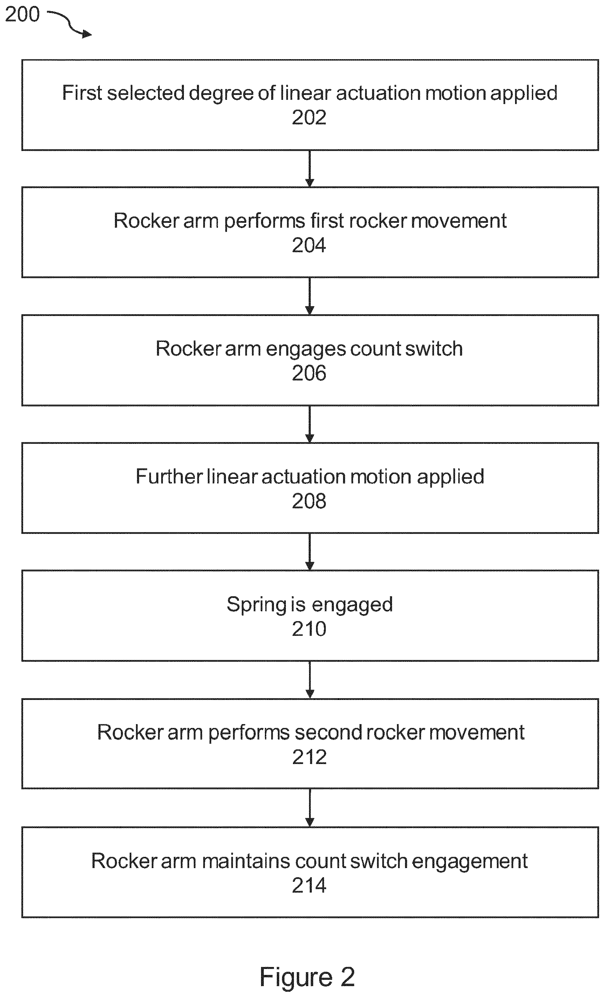

Figure 2 shows a schematic flow diagram of a method of use of an electronic inhaler counter 200, for example the electronic inhaler counter described in Figure 1 or Figures 3A to C. Starting at step 202, a first selected degree of linear actuation motion is applied to the electronic inhaler counter 100. This displaces the rocker arm 102 relative to the inhaler actuator tongue 404, until the rocker section 116 of the rocker arm 102 contacts the actuator tongue 404. In the example shown in Figure 3B, the contact between the rocker section 116 of the rocker arm 102 and the actuator tongue 404 causes the rocker arm 102 to perform a first rocker movement at step 204, wherein the pivot 104 is held down by the force of the pre-compressed spring 108 and the rocker section 116 of the rocker arm pivots the rocker arm 102 until the head 114 of the rocker arm 102 engages the count switch 106 at step 206. In the example shown in Figure 3B, the pivot 104 is held down and the rocker arm 102 pivots in an anti-clockwise rotation until the rocker arm 102 engages the count switch 106 at step 206. In some examples, only a first degree of linear actuation is applied, and the method ends. In some examples, further linear actuation motion is applied to the electronic inhaler counter 100 at step 208. The spring 108 then engages at step 210. In the example shown in in Figure 3C, the spring 108 engages by compressing. The spring 108 engaging causes the rocker arm 102 to perform a second rocker movement at step 212. In some examples, the second rocker movement is in the opposite direction to the first rocker movement. In the example shown in Figure 3C, the spring 108 compresses and lifts the pivot 104, this causes the rocker arm 102 to pivots about the rocker section 116 in contact with the actuator tongue 404 in a clockwise rotation. In the example shown in Figure 3C, the head 114 of the rocker arm 102 maintains engagement with the count switch 106 at step 214. In some examples, the head 114 of the rocker arm 104 may disengage from the count switch 106 during further linear actuation.

Figure 3A shows an example schematic illustration of an example electronic inhaler counter prior to an actuation event. Figure 3B illustrates the first rocker movement 204 of the rocker arm 102 during an actuation event. Figure 3C illustrates the engagement 210 of the spring 108 and the second rocker movement 212 of the rocker arm 102 during an actuation event.

Figure 3A shows an example of an electronic inhaler counter 100. The electronic inhaler counter 100 consists of four moulded parts: a two-shot housing 120 with a user button, a two-shot bezel 122 with a clear window, a rocker arm 102, and a spring retainer cap 118. In this example, the housing 120, bezel 122, and spring retainer cap 118 are made of polybutylene terephthalate (PBT), however in other examples other materials can be used. In this example, the clear window is made of polycarbonate, however in other examples other clear materials can be used. In this example, the rocker arm is made is acetal, however in other examples other materials can be used.

The electronic inhaler counter 100 shown in Figures. 3A to 3C further comprises a spring 108, a battery 128, and a PCBA 124. In some examples, the electronic inhaler counter 100 additionally comprises a soft pad. In the example shown, the electronic inhaler counter 100 comprises a soft pad made of poron foam, however in other examples other materials can be used. The soft pad may be coupled to the PCBA 124 to protect the circuitry from compressive forces during linear actuation cycles.

Within the housing 120, the electronic inhaler counter 100 of comprises the rocker arm 102, the rocker arm 102 comprising a proximal end 110 providing a pivot 104 and a distal end 112 providing a head 114. The rocker arm 102 further comprises a rigid rocker section 116. In the example shown, the rocker section 116 has a dogleg shape. In other examples, the rocker section 116 can have other curved shapes or geometries to provide a pivot.

The count switch 106 is arranged above the rocker arm head 114. The count switch 106 is coupled to the underside of a printed circuit board assembly (PCBA) 124.

In this example, the battery 128 is a coin battery, for example a CR2032 battery, however in other examples other batteries or power supplies can be used. The battery 128 is coupled to the upper surface of the PCBA 124. In this example, custom battery clips are used. The clips are surface mounted, for example by reflow soldering, onto of the PCBA 124 and consist a c-clip (positive terminal) and a cross-shaped contact (negative terminal). This design may help to keep the form factor of the electronic inhaler counter 100 within the envelope of the mechanical inhaler counter (for example such as that disclosed in WO 2006/110080) and to retain the overall height. The battery 128 is retained in the vertical direction by the bezel 122 and the stack of components above the battery. The cross-shaped clip comprises two sprung arms configured to flex and provide sufficient contact force through the range of movement anticipated in the vertical direction. The surface mounted c-clip restrains the battery 128 along the plane of the PCBA 124 and has 4 legs that are reflow soldered to the PCBA 124. The inner clip legs make electrical contact to the board while optional additional outer clip legs serve a purely mechanical function.

The return spring 108 is arranged such that the axis of compression is parallel to the direction of linear actuation. One end of the spring 108 is coupled to the rocker arm pivot 104. In this example, the other end of the spring 108 is retained by a spring retainer cap 118.

An advantage of the retainer cap 118 may be to allow a longer spring to be used in the device than would otherwise be possible, hence allowing further overtravel and the ability to provide the required force without reaching solid height. In other examples not comprising a spring retainer cap 118, the end of the spring 108 not coupled to the pivot 104 may be coupled to the underside of the PCBA 124, or otherwise directly coupled to the housing.

The spring retainer cap 118 that houses the spring is arranged to allow the spring to extend beyond the PCBA 124 and is configured to maximize the space available for the spring 108. In this example, the spring retainer cap 118 is a push fit into the housing 120. In other examples, the spring retainer cap may be retained by other means. As a failsafe against it coming loose, the movement of the spring retainer cap 118 is restricted by the PCBA 124 above it. The movement in the PCBA 124, in turn, is restricted by legs protruding downwards from the bezel 122.

In this example, a helical coil spring 128 is used which includes 3 dead coils in the middle to reduce the possibility of spring tangling during assembly. In other examples, other numbers of dead coils may be used within the spring 128. Once the electronic inhaler counter 100 is assembled, the spring 128 is pre-compressed to a height set by the space available in the electronic inhaler counter 100. In this example the spring 128 is pre-compressed to a height of 10.9 mm.

In the example shown in Figure 3, the digital display arrangement is provided in a top surface of the counter housing 120. In the disclosed embodiment, the top surface of the housing is provided as a transparent moulded window that closes the housing, retained by the bezel 122. In some examples, the counter top surface further is utilized as an actuating surface for actuation of the linear actuation motion, i.e. for depressing the container-counter assembly. Because the counter top surface is used as actuating surface, it is configured to be rigid and wear resistant, as it will be subjected to compressive force and wear during the actuation of the inhaler device.

The electronic inhaler counter 100 is arranged wherein, in response to a first selected degree of linear actuation motion, the rocker arm 102 is configured to perform a first rocker movement and engage the count switch 106 with the rocker head 114; and in response to further linear actuation motion, the spring 108 is engaged to enable the rocker arm 102 to perform a second rocker movement.

In response to the removal of biasing force, the spring 108 is configured to return to its original configuration, also returning the rocker arm 102 to its original configuration.

In this example, the electronic inhaler counter 100 is configured to attach to the end of an inhaler container (not shown), mounted in an inhaler actuator housing 402, wherein the linear actuation motion is relative to the actuator housing 402 and the rocker arm 102 is configured to engage with an actuator tongue 404 coupled to the actuator housing 402.

In this example, the rocker section 116 of the rocker arm 102 is configured to engage with the actuator tongue 404 of an inhaler device. In this example, the actuator tongue 404 is arranged to protrude through an opening in the counter housing 120 to engage the rocker arm 102.

In some examples, the first selected degree of linear actuation motion, as shown in Figure 3B, engages a first portion of the rocking section 116 of the rocker arm 102 with the actuator tongue 404 to displace the rocker arm 102 in a first direction to engage the rocker head 114 with the count switch 106. In some examples, the second selected degree of linear actuation motion engages a second portion of the rocker section 116 of the rocker arm 102 with the actuator tongue 404 to rock the rocker arm 102 in a second direction, as shown in Figure 3C.

In the example shown, as the counter 100, attached to the container, is depressed by the user by the linear actuation motion relative to the actuator housing 402, it reaches the fire point after 2.07 mm of container valve travel, however this may vary based on container valve component tolerances. In use, the electronic inhaler counter 100 registers a count early in the container stroke/linear actuation cycle. In the example shown, the target separation between the count point and the fire point is 0.78 mm, which, from the tolerance analysis, yields a probability of fire before count of 8 ppm.

In other examples, the count-fire separation and the standard deviation on it will depend on the stack of tolerances through the chain and the uncertainty associated with the smart assembly process. The largest contributors in this case are external to the electronic inhaler counter 100, for example the positional uncertainty of the actuator tongue 404 relative to the electronic inhaler counter 100 through the container and actuator body 402 (standard deviation of 0.15 mm). The main contributors there are the uncertainties associated with the smart assembly height and the container stroke to count.

In setting the count point early in the travel, the electronic inhaler counter 100 must be guarded against inadvertent counting where small displacements of the container would be registered as a count. In the count-fire separation in the example shown, the chance of a device counting at displacements approaching zero is calculated to be extremely small (of the order of 10⁻¹³).

When the first rocker movement is actuated by an actuator tongue 404 on the inhaler actuator body 402, the pivot point 104 must thus be held down. However, the device 100 must also allow significant over travel, hence the pivot 104 is held down by the pre-compressed spring 108. The spring 108 can be further compressed to allow significant over travel but is sufficiently stiff that the pivot 104 does not move until the count button has registered a count in response to a first selected degree of linear actuation motion.

In this example, the nominal force to hold down the pivot at switching during the first selected degree of linear actuation motion is calculated at 0.77 N but may be up to 1.27 N at 5 standard deviations. Therefore, in some examples, 1.27 N is set as the requirement for the minimum force the spring must be able to provide once pre-compressed.

Beyond button actuation, the mechanism needs to support overtravel while not significantly increasing (< 5N increase) the user force requirement to fire the container. In this example, the nominal extra force the spring provides is 3.13 N at container fire and 5 N at maximum canister overtravel.

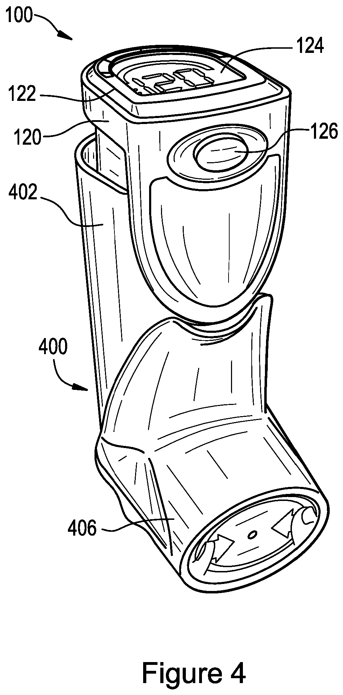

Figure 4 shows a schematic example of an inhaler device 400 comprising an electronic inhaler counter 100 according to the present invention. The inhaler device comprises an actuator body 402 with a mouth-piece, through which medicine is delivered to the user, and a container-counter assembly. In this example, the mouth-piece is covered by a mouth piece cover 406, configured to attach to the mouth piece when not in use for hygiene considerations. In this example, the counter 100 is attached to the end of an inhaler container (not shown) arranged in the actuator housing 402. The counter 100 may attached to the inhaler container in an assembling process and in other examples it can be attached to the inhaler container at any one of numerous points along the canister end opposite the valve, i.e. the part of the canister opposite from the valve stem, from the outermost edge of the counter 100 to its inside base giving ranges of variation of positions and varying lengths of canister tolerances, i.e. the counter can be attached anywhere on the base of the canister. The counter 100 could further be arranged as a part of, or being detachably attached to the actuator housing 502, e.g. on the front or back side thereof.

The inhaler device 400 is actuated by depressing the container-counter assembly with respect to the actuator housing 402. The counter 100 is arranged to count each actuation of the inhaler device 400, and display the actual condition, via a display arrangement 124.

The example of the electronic inhaler counter 100 shown in Figure 4 comprises a digital display 124. In this example, the display arrangement 124 is provided in a top surface of the counter housing 120. In this example, the digital display 124 provides the absolute number of linear actuations/doses of the inhaler device remaining and a relative indication of the number of linear actuations/doses of the inhaler device remaining. In this example, the relative indication is provided as a semi-annular bar which reduces in length in graduations proportional to the number of doses remaining in the inhaler device. In this example, the display arrangement 124 further comprises a static portion of the display, on the bezel 122. In this example, the relative indication of the number of linear actuations/doses of the inhaler device remaining may be compared to the static portion of the display. In this example, the static portion of the display is a semi-annular graduated area. In some examples, at least a portion of the graduated area may be colour-coded. In this example, two portions of the graduated area are colour coded to indicate when the relative number of linear actuations/doses of the inhaler device remaining is getting low (in this example indicated in yellow) and critically low (in this example indicated in red). In other examples, the graduated area may include other colour-coded systems. In other examples, the graduated area may include indices for the number of doses remaining in the container.

The example of the electronic inhaler counter shown 100 in Figure 4 further comprises a user button 126, disposed on the outer surface of the housing 120. In some examples, the user button 126 may be used to switch on/off the digital display 124. In some examples, the user button 126 may be configured to "wake" the digital display 124. In some examples, this may help to extend the battery life of the electronic inhaler counter 100 as the display 124 may be turned off or enter a "sleep" mode to preserve battery life during periods of non-use. In some examples, the "sleep" mode may be activated after a predetermined time of non-use. In some examples, the user button 126 may be configured to switch between display interfaces on the digital display 124. For example, the user button 126 may enable to user to switch between a display showing the number of doses remaining, and a display showing the number of linear actuations/doses recorded. In some examples, the user button may be configured to activate Bluetooth pairing of the device. In some examples, the user button may be configured to activate Bluetooth pairing of the device when depressed for a certain time period i.e. press and hold. In some examples, the user button may be configured to have different functionality in response to a press actuation and a press-hold actuation.

### Inhaler breath detection module

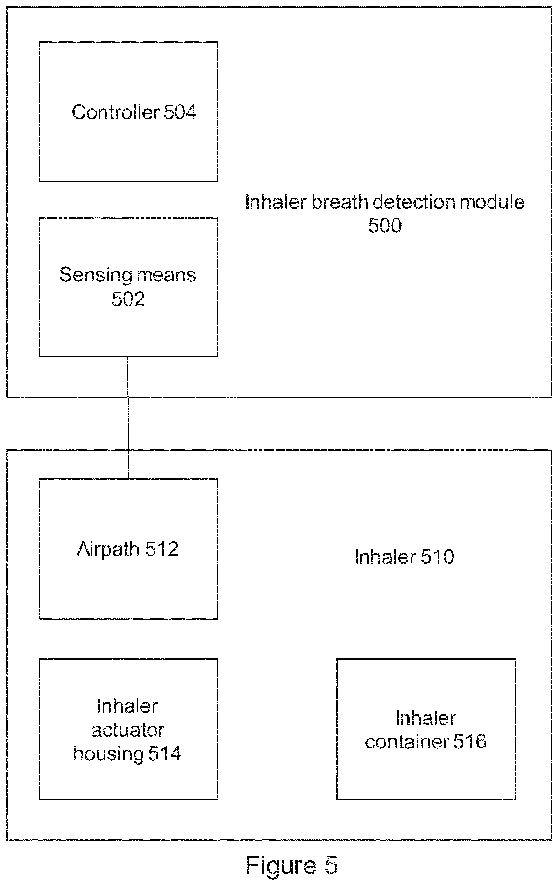

Figure 5 shows an example inhaler breath detection module 500 for detecting the beginning and/or end of an inhalation breath. The breath detection module 500 is coupled to an airpath 512 of the inhaler 510, and comprises a sensing means 502 and a controller 504.

The sensing means 502 is configured to provide signals indicative of a change in parameter in the inhaler airpath 512 as a function of time. The controller 504 is configured to receive the signals indicating of a change in parameter in the inhaler airpath 512 as a function of time from the sensing means 502.

In use, an inhalation breath taken by a user during operation of an inhaler causes a change in parameter in the inhaler airpath 512. The controller 504 is configured to determine the presence of breath, based on a change in parameter in the inhaler airpath 512 as a function of time.

In some examples, the sensing means 502 may comprise at least one pressure sensor. The pressure inside the device will be lower than outside during an inhalation, thus by measuring the difference in pressure, a flow rate may be calculated, and the presence of a breath may be detected. In some examples, at least one differential pressure sensor may be used with one port connected to an appropriate location inside the device and with the other port open to atmosphere. In some examples, at least one absolute pressure sensor may be used. In some examples, the at least one absolute pressure sensor may be a miniature board mount barometric pressure sensor, for example a MS5607 barometric pressure sensor. In a first configuration, two absolute pressure sensors may be used where one measures atmospheric pressure and the other pressure inside the device. In a second configuration, a single absolute sensor may be used that measures pressure inside the device and tracks changes in pressure over time. The second configuration may have reduced cost and form factor implications. However, the second configuration may require compensation for non-breath changes in pressure such as attitude changes and barometric pressure variations. Additionally, all pressure sensor approaches require a sealed pathway to the correct measurement location.

In some examples, the sensing means 502 may comprise at least one microphone. In some examples, the at least one microphone may be a digital microphone. In some examples, the at least one microphone may be an analogue microphone, further comprising a pre-amplifier circuit and an analogue-to-digital converter (ADC). An advantage of a digital microphone may be a reduction and cost relative to an analogue microphone including the pre-amplifier circuit and ADC. A microphone may be used to record sound generated as air flows through an acoustic feature coupled to the inhaler airpath. A machine learning algorithm may then be used to determine the presence of breath (logistic regression) and compute flow rate (linear regression). The use of a microphone may overcome the form factor and cost constraints associated with pressure sensors, as well as the requirement for a reliably sealed port.

However, unlike pressure sensors, the signal may be prone to error from noise and is a less direct measure of flow rate.

In some examples, the inhaler breath detection device may comprise a second microphone. A second microphone may be used to achieve a 'noise cancelling' function by recording the ambient noise far from the inhaler cavity. This could provide improved breath detection accuracy in noisy environments.

In some examples, the sensing means 502 may comprise other sensing means to detect flow rate by, for example, electromagnetic, optical, mechanical, and/or thermal methods. For the most accurate measurement, a flow rate sensor would require all the flow to be diverted through it and a fundamental change to the flow path and flow resistance of existing pMDI devices. An alternative approach would be to bleed some of the flow through the flow rate sensor and correlate the readings to the overall flow rate. This, however, would be highly sensitive to the relative flow resistances through the flow meter and the main flow path, as well as to the relative amount of air bled. It would also require a well-sealed pathway to and from the flow rate sensor.

For pMDls, lower flow rates are generally seen as beneficial in literature as they promote better deposition of drug into the lung. A study was carried out on the inspiratory flow rates through pMDIs in practise, considering both untrained patients and those trained to inhale correctly (more slowly) through a pMDI. The results demonstrate that users achieve a range of inspiratory flow rates but that in all cases the flow rates are above 30 I/min. Therefore, in some examples, the sensing means may be configured to detect a lower flow rate detection limit of 30 I/min.

In some examples, the inhaler breath detection module 500 further comprises an acoustic feature. An acoustic feature may be configured to alter a property of the airflow sensed by the sensing means. In some examples, an acoustic feature may be configured to alter a property of the airflow such that the signal recorded by the sensing means is amplified. This may reduce the susceptibility to noise and increase the signal to noise ratio for more accurate breath and flow rate detection at low flow rates.

In some examples, the acoustic feature comprises a narrowing or restriction through which a portion of the airflow is configured to flow when a user takes a breath. In some examples, the acoustic feature comprises a narrowing or restriction of the distance between the inhaler breath detection module and the inhaler actuator housing.

In order to amplify the signal recorded by the microphone and increase the signal to noise ratio, various whistle generating mechanisms were identified from literature and prototyped and tested to characterise their performance. While all implementations produced some improvement over the baseline, the largest improvement was produced by the introduction of a small side orifice that creates jet noise near the microphone. At low flow rates the increase is of the order of 3x, while at higher flow rates, 10x amplification is achieved. Thus, in some examples, the acoustic feature may comprise a side orifice, configured to increase the inferred sound pressure at the microphone.

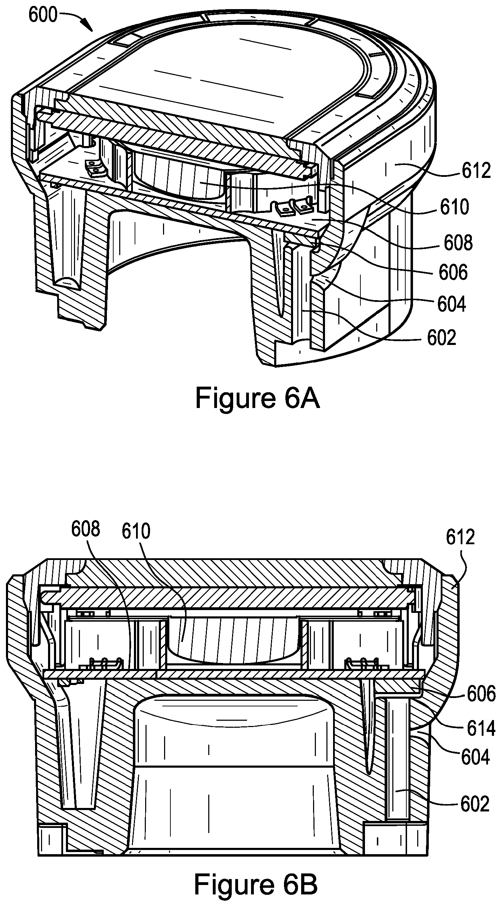

Figures 6A and 6B show an example wherein the inhaler breath detection module is mounted into the housing 612 of the electronic inhaler counter 600. By locating the inhaler breath detection module within the electronic inhaler counter housing 612, this may provide an advantage by not increasing the form factor of the device. In some examples, the housing 612 is has the same footprint as the cavity housing of the original mechanical counter disclosed in WO 2006/110080.

The microphone 606 sits over a port 614, coupled to a narrow channel 602, arranged parallel to the longitudinal axis. The channel 602 couples the microphone 606 to the airpath of the inhaler. A side orifice 604 is arranged within the channel 602, close to the microphone 606. The side orifice 604 is coupled to the airpath of the inhaler. The microphone 606 is further coupled to a printed circuit board assembly (PCBA) 608, comprising a controller. A coin battery 610 is also coupled to the upper side of the PCBA 608. The PCBA 608 and battery 610 may be configured for dual-use by the breath detection module and electronic inhaler counter 600.

The side orifice 604 is an example of an acoustic feature and is configured to increase the sound pressure at the microphone, thus amplifying the microphone signal.

In use, flow from the airpath enters through the orifice 604 and generates turbulent noise in the vicinity of the microphone 606, for example a jet noise or whistling sound. The airflow is then diverted down the channel 602z. In other examples, the acoustic feature may otherwise comprise any other narrowing or restriction through which a portion of the airflow is configured to flow.

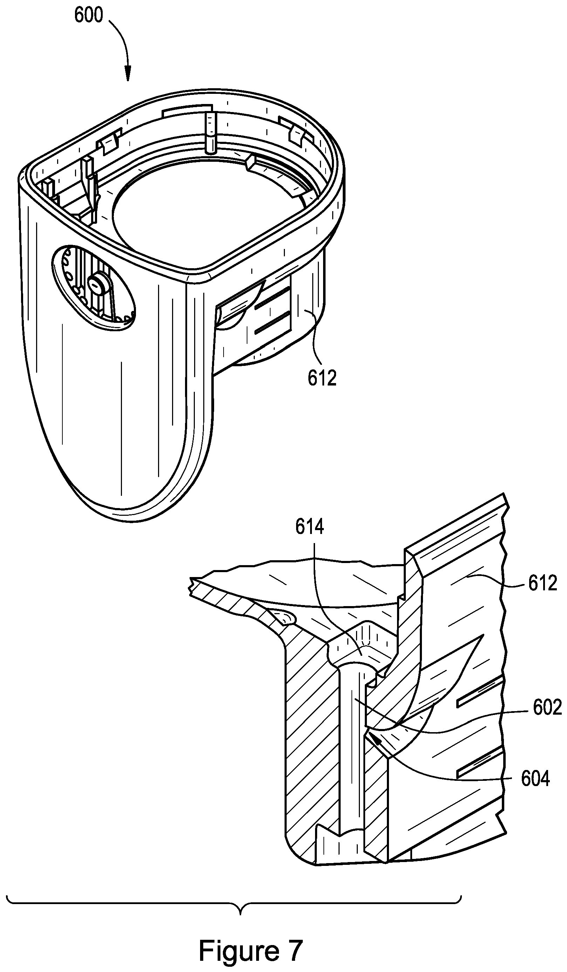

Figure 7 shows a detailed section of the example breath detection device of Figure 6 mounted into the housing 612 of the electronic inhaler counter 600. The housing 612 may be configured to attach to an inhaler device. In the example shown, the inhaler breath detection module is configured to attach to the end of an inhaler container mounted in an inhaler actuator housing, for example as depicted in Figure 4 by housing 120. In the section view, the microphone (not shown) sits over a port 614 which is coupled to the main airpath through the small side orifice hole 604 in the housing body 612. In this example, the side orifice 604 has a minimum diameter of 0.4 mm.

In use, flow from the main airpath may enter through the orifice 604 and generate turbulent noise in the vicinity of the microphone. The flow is then diverted down the channel 602.

The correlation of microphone reading to flow rate is not as straightforward as pressure readings. A relationship between signal power and flow rate can be empirically obtained and the signal processing can be manually optimised for the range of frequencies of interest. However, the relationship is likely to change in different noise environments and optimisation would require tuning in each setting.

In some examples, a machine learning approach may be implemented whereby the signal from the microphone is processed by the controller using pre-trained parameters. An advantage of using a machine learning approach may be to improve the determination of the relationship between the microphone sensing signal and flow rate, particularly in different noise environments. This may improve the accuracy of flow rate and breath determination in different noise environments. In some examples, the machine learning algorithm may be trained on inhalation samples and noise samples across a range of flow rates. In some examples the training parameters relate power at each frequency to different weightings. In some examples, the machine learning implementation performs logistic regression which outputs a confidence level of the presence of breath. In some examples, the machine learning implementation performs linear regression which outputs an estimate of flow rate. In some examples, the machine learning implementation performs both logistic regression and linear regression. In some examples, the output data can be used to detect when a breath starts and/or ends.

In some examples, the output generated by the machine learning implementation is subject to post-processing, for example, filtering. In some examples, Gaussian filters can be applied to smooth out the data, and/or morphological filters can be applied to remove outlier "blips". In some examples, all post processing and filtering may be performed on a remote device, for example on an app.

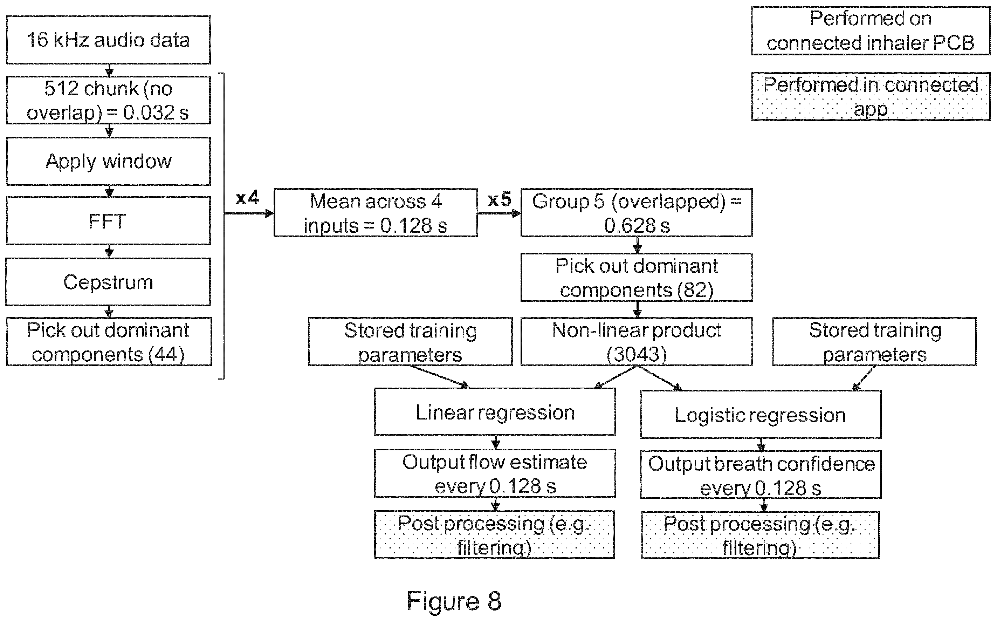

In one example, Figure 8 provides an overview of how data may be handled by the algorithm. The digital microphone outputs 16 kHz audio data which is broken into 512 bit chunks (equivalent to 0.032 s). This data is run through two transforms - first a fast Fourier transform (FFT) to obtain the spectrum and then an inverse Fourier transform on the logarithm of the spectrum to obtain the cepstrum. The cepstrum may contain information about the rate of change in different spectrum bands and is common in speech analysis as it is useful in picking out the base frequencies and harmonics. In this example, a principle component analysis is carried to pick out the 44 top dominant components. The average of these components across four consecutive 512 bit chunks of audio input is then taken representing 0.128 s of data. Next, five such averages are grouped together for further processing (comprising 0.628 s of data) where each successive group of five averages has 0.500 s overlap with the previous group (i.e. there is a shift of 0.128 s, or one average, from group to group).

Continuing with the example shown in Figure 8, principal component analysis to pick out the dominant components may be carried out on each successive group followed by an operation to compute the non-linear product. It is this non-linear product that constitutes the feature set on which the algorithm separately carries out linear regression (flow rate estimate) and logistic regression (breath detection) based on stored training parameters. The algorithm, thus, outputs an estimate of flow rate and an estimate of the confidence of presence of breath every 0.128s equivalent to the time shift between successive groups of data. The estimates output by the machine learning algorithm may be transferred to a connected remote device (e.g. cell phone) where post processing (e.g. filtering of the data to smooth it) can be carried out. In some examples, all the steps outlined above carried out on the electronic inhaler counter PCB and the conditioning steps of grouping, averaging and principle component analysis may be necessary to "compress" the data to a manageable size for the controller, for example a PCB microcontroller.

In some examples, the inhaler breath detection module may further comprise an accelerometer. In some examples, the accelerometer may be configured to detect the orientation of the device. In some examples, the accelerometer may be configured to detect shaking of the device. A shake may be defined by a change in acceleration greater than a pre-determined threshold rate of change. In some examples, the inhaler breath detection module may record shake data prior to a puff, for example 5 seconds prior to a puff. Recording shaking of the device may be advantageous for providing data on the priming of the device prior to an actuation event. For example, in some examples the inhaler breath detection module may be configured to detect the presence of priming/shaking prior to an actuation event. In some examples, the inhaler breath detection module may be configured to record the duration or intensity of priming/shaking.

### Feedback

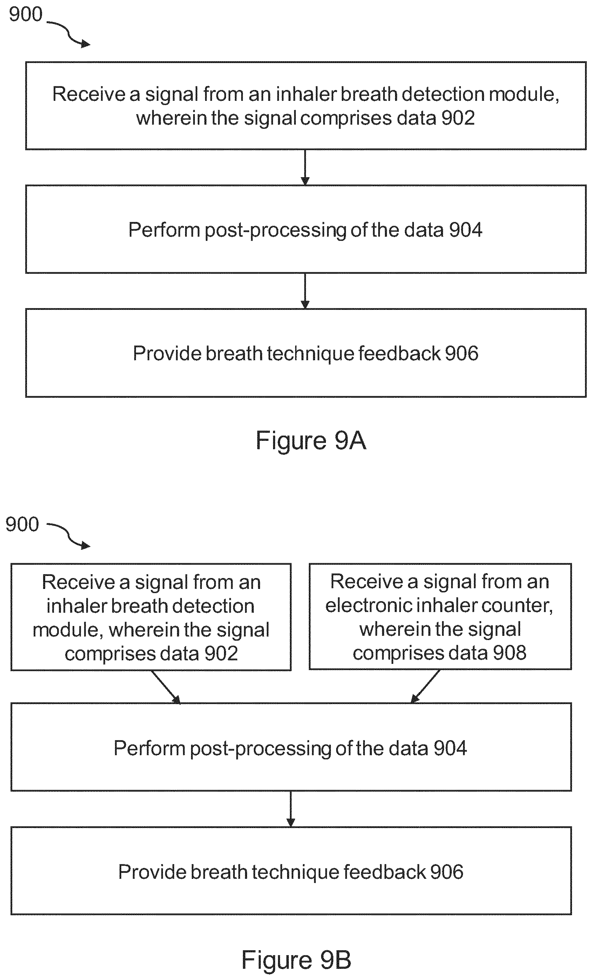

Figure 9A shows a schematic flow diagram of a method 900 of signal processing and analytics by an inhaler technique feedback app. Firstly, the app receives a signal from an inhaler breath detection module, wherein the signal comprises data, at step 902. The data may include at least one of a confidence level of breath, and/or an estimate flow rate of breath.

At step 904, the app performs post-processing of the data. This step may involve filtering of the signals. In some examples, the post-processing of data includes filtering, for example using Gaussian filters or morphological filters. In some examples, filtering can be used to smooth out the data, and/or to remove outlier "blips". In some examples post-processing may also include various pruning routines, rule quality processing, rule filtering, rule combination, model combination, or knowledge integration.

In some examples, the signal received by the inhaler technique feedback app from the inhaler breath detection module further comprises other data, for example breath duration, time stamp data of breath, and/or shake data.

At step 906, the app is configured to provide breath technique feedback, based on the at least one of the confidence level of breath, and/or an estimate flow rate of breath relative to predetermined optimal ranges of use.

In some examples, the app is configured calculate the breath duration using the confidence level of breath. For example, the app is configured to calculate the breath duration using the confidence level of the start and end of a breath.

In some examples, the app may be configured to detect if a breath is longer than a predetermined time period, for example 2 s. In some examples, the app may be configured to detect is a breath has been taken after firing. In some examples, the app may be configured to detect if breath flow rate is maintained below a predetermined rate, for example 100 I/min. In some examples, the app may be able to detect if no breath is associated with an actuation. In some examples, the app may be able to detect if no breath-hold is associated with an actuation.

In some examples, the inhaler technique feedback app may be configured to provide technique feedback based on aggregated data and/or long-term use patterns. This may help the user address potential reoccuring modes of inhaler misuse, for example, incorrect timing of delivery/breath, not inhaling, sub-optimal inhalation flow profile (i.e. too fast or too slow), and/or no breath-hold after inhaling.

As shown in Figure 9B, in some examples, the inhaler technique feedback app may be further configured to receive data from an electronic inhaler counter, wherein the signal comprises data, at step 908. In some examples, the data may include at least one of the dose count and time stamp data relating to a dose count, and/or the fire point and time stamp data relating to a fire point. In some examples, the system may use recorded data about a fire point of the inhaler, example the time stamp data, to determine the timing of a puff relative to the start and/or end of a breath. This may be used to provide feedback to the user on the timing of delivery/breath, for example if the timing of the breath was outside a pre-determined time range relative to the timing of inhaler actuation, for example, if breath was too early or too late relative to inhaler actuation.

In some examples, the inhaler technique feedback app may be further configured to provide an indication to the user of how many doses have been administered in a certain time period, for example, in a day. In some examples, the inhaler technique feedback app may be further configured to provide an indication to the user about what time at least one dose was administered. This may help the user track their dose history, important for effective management of the user's treatment and/or condition.

In some examples, the inhaler technique feedback app may be further configured to receive shake data and/or orientation data of the device from the breath detection module. In some examples, the system may use recorded data about a fire point or dose count of the inhaler, for example the time stamp, to determine the shake technique and/or timing of a puff relative to a shake. This may be used to provide feedback to the user on the shaking technique prior to a puff.

In some examples, the system may receive data after every actuation event. In some examples, the system may receive data for each actuation event during a batch download.

## Claims

1. An electronic inhaler counter (100) for counting linear actuation of a pressurised metered dose inhaler, pMDI, the electronic inhaler counter (100) comprising:
a rocker arm (102) comprising a proximal end (110) providing a pivot (104) and a distal end (112) providing a head (114);
a return spring (108) coupled to the rocker arm pivot (104); and
a count switch (106);
wherein, in response to a first selected degree of linear actuation motion, the rocker arm (102) is arranged to perform a first rocker movement and engage the count switch (106) with the rocker head (114); and in response to further linear actuation motion, the spring is engaged to enable the rocker arm (102) to perform a second rocker movement, such that the rocker head (114) maintains engagement with the count switch (106).

2. The electronic inhaler counter of claim 1 wherein the electronic inhaler counter (100) is configured to attach to the end of an inhaler container mounted in an inhaler actuator housing (402), and wherein the linear actuation motion is relative to the actuator housing and the rocker arm (102) is configured to engage with an actuator tongue (404) coupled to the actuator housing (402).

3. The electronic inhaler counter of claim 2 wherein the first selected degree of linear actuation motion engages a first portion of the rocker arm (102) with the actuator tongue (404) to rock the rocker arm (102) in a first direction to engage the rocker head (114) with the count switch (106), and wherein the second selected degree of linear actuation motion engages a second portion of the rocker arm (102) to rock the rocker arm (102) in a second direction to maintain engagement of the rocker head (114) with the count switch (106).

4. The electronic inhaler counter of any of the previous claims wherein the first rocker movement is an anti-clockwise rotation about the pivot (104), and the second rocker movement is a clockwise rotation about the count switch (106).

5. The electronic inhaler counter of any of the previous claims wherein the first selected degree of linear actuation motion does not compress the return spring (108), and wherein the second selected degree of linear actuation motion compresses the return spring (108).

6. The electronic inhaler counter of claim 2 wherein at least a portion of the inhaler breath detection module is configured to fit within at least a portion of the inhaler actuator housing (402).

7. The electronic inhaler counter claim 2 or 5 wherein the inhaler device is actuated by depressing the electronic inhaler counter (100) with respect to the actuator housing (402).

8. The electronic inhaler counter of claim 6 wherein when the inhaler device is actuated at least a portion of the electronic inhaler counter (100) fits within the inhaler actuator housing (402).

9. The electronic inhaler counter of any of the previous claims, further comprising a digital display (124), wherein the display is configured to display at least one of the counted number of linear actuations of the inhaler device, and/or the number of linear actuations of the inhaler device remaining, wherein the number of linear actuations of the inhaler device remaining is calculated by the counted number of linear actuations of the inhaler device subtracted from a predetermined maximum number of linear actuations of the inhaler device.

## Patentansprüche

1. Elektronischer Inhalatorzähler (100) zum Zählen linearer Betätigungen eines treibgasbetriebenen Dosierinhalators, pMDI, wobei der elektronische Inhalatorzähler (100) Folgendes umfasst:
einen Kipphebel (102), der ein proximales Ende (110) mit einem Drehzapfen (104) und ein distales Ende (112) mit einem Kopf (114) umfasst;
eine Rückstellfeder (108), die mit dem Kipphebeldrehzapfen (104) gekoppelt ist; und
einen Zählschalter (106);
wobei der Kipphebel (102) so angeordnet ist, dass er als Reaktion auf einen ersten ausgewählten Grad einer linearen Betätigungsbewegung eine erste Kippbewegung ausführt und den Zählschalter (106) mit dem Kippkopf (114) in Eingriff bringt; und als Reaktion auf eine weitere lineare Betätigungsbewegung die Feder in Eingriff gebracht wird, um dem Kipphebel (102) eine zweite Kippbewegung derart zu ermöglichen, dass der Kippkopf (114) weiterhin mit dem Zählschalter (106) in Eingriff bleibt.

2. Elektronischer Inhalatorzähler nach Anspruch 1, wobei der elektronische Inhalatorzähler (100) konfiguriert ist, um am Ende eines Inhalatorbehälters befestigt zu werden, der in einem Inhalatorbetätigergehäuse (402) montiert ist, und wobei die lineare Betätigungsbewegung relativ zum Betätigergehäuse erfolgt und der Kipphebel (102) konfiguriert ist, um mit einer Betätigerzunge (404), die mit dem Betätigergehäuse (402) verbunden ist, in Eingriff zu kommen.

3. Elektronischer Inhalatorzähler nach Anspruch 2, wobei der erste ausgewählte Grad der linearen Betätigungsbewegung einen ersten Abschnitt des Kipphebels (102) mit der Betätigerzunge (404) in Eingriff bringt, um den Kipphebel (102) in eine erste Richtung zu kippen, um den Kipphebelkopf (114) mit dem Zählschalter (106) in Eingriff zu bringen, und wobei der zweite ausgewählte Grad der linearen Betätigungsbewegung einen zweiten Abschnitt des Kipphebels (102) in Eingriff bringt, um den Kipphebel (102) in eine zweite Richtung zu kippen, um den Eingriff des Kipphebelkopfs (114) mit dem Zählschalter (106) aufrechtzuerhalten.

4. Elektronischer Inhalatorzähler nach einem der vorstehenden Ansprüche, wobei die erste Kippbewegung eine Drehung gegen den Uhrzeigersinn um den Drehzapfen (104) ist und die zweite Kippbewegung eine Drehung im Uhrzeigersinn um den Zählschalter (106) ist.

5. Elektronischer Inhalatorzähler nach einem der vorstehenden Ansprüche, wobei der erste ausgewählte Grad der linearen Betätigungsbewegung die Rückstellfeder (108) nicht zusammendrückt und wobei der zweite ausgewählte Grad der linearen Betätigungsbewegung die Rückstellfeder (108) zusammendrückt.

6. Elektronischer Inhalatorzähler nach Anspruch 2, wobei mindestens ein Abschnitt des Atemerkennungsmoduls des Inhalators konfiguriert ist, um in mindestens einen Abschnitt des Inhalatorbetätigergehäuses (402) zu passen.

7. Elektronischer Inhalatorzähler nach Anspruch 2 oder 5, wobei die Inhalatorvorrichtung durch Niederdrücken des elektronischen Inhalatorzählers (100) in Bezug auf das Betätigergehäuse (402) betätigt wird.

8. Elektronischer Inhalatorzähler nach Anspruch 6, wobei bei Betätigung der Inhalatorvorrichtung mindestens ein Abschnitt des elektronischen Inhalatorzählers (100) in das Gehäuse des Inhalatorbetätigerelements passt (402).

9. Elektronischer Inhalatorzähler nach einem der vorstehenden Ansprüche, ferner umfassend eine digitale Anzeige (124), wobei die Anzeige konfiguriert ist, um mindestens die gezählte Anzahl linearer Betätigungen des Inhalators und/oder die verbleibende Anzahl linearer Betätigungen der Inhalatorvorrichtung anzuzeigen, wobei die verbleibende Anzahl linearer Betätigungen der Inhalatorvorrichtung berechnet wird, indem die gezählte Anzahl linearer Betätigungen der Inhalatorvorrichtung von einer vorgegebenen maximalen Anzahl linearer Betätigungen der Inhalatorvorrichtung abgezogen wird.

## Revendications

1. Compteur électronique d'inhalateur (100) permettant de compter un actionnement linéaire d'un inhalateur doseur sous pression, pMDI, le compteur électronique d'inhalateur (100) comprenant :
un bras basculant (102) comprenant une extrémité proximale (110) fournissant un pivot (104) et une extrémité distale (112) fournissant une tête (114) ;
un ressort de rappel (108) accouplé au pivot (104) de bras basculant ; et
un commutateur de comptage (106) ;
dans lequel, en réponse à un premier degré sélectionné de mouvement d'actionnement linéaire, le bras basculant (102) est agencé pour mettre en œuvre un premier déplacement basculant et mettre en prise le commutateur de comptage (106) avec la tête basculante (114) ; et en réponse à un mouvement d'actionnement linéaire supplémentaire, le ressort est en prise pour permettre au bras basculant (102) de mettre en œuvre un second déplacement basculant, de telle sorte que la tête basculante (114) maintient une mise en prise avec le commutateur de comptage (106).

2. Compteur électronique d'inhalateur selon la revendication 1 dans lequel le compteur électronique d'inhalateur (100) est conçu pour se fixer à l'extrémité d'un récipient d'inhalateur monté dans un logement d'actionneur (402) d'inhalateur, et dans lequel le mouvement d'actionnement linéaire est par rapport au logement d'actionneur et le bras basculant (102) est conçu pour venir en prise avec une languette d'actionneur (404) accouplée au logement d'actionneur (402).

3. Compteur électronique d'inhalateur selon la revendication 2 dans lequel le premier degré sélectionné de mouvement d'actionnement linéaire met en prise une première partie du bras basculant (102) avec la languette d'actionneur (404) pour basculer le bras basculant (102) dans une première direction pour mettre en prise la tête basculante (114) avec le commutateur de comptage (106), et dans lequel le second degré sélectionné de mouvement d'actionnement linéaire met en prise une seconde partie du bras basculant (102) pour basculer le bras basculant (102) dans une seconde direction pour maintenir une mise en prise de la tête basculante (114) avec le commutateur de comptage (106).

4. Compteur électronique d'inhalateur selon l'une quelconque des revendications précédentes dans lequel le premier déplacement basculant est une rotation en sens antihoraire autour du pivot (104), et le second déplacement basculant est une rotation en sens horaire autour du commutateur de comptage (106).

5. Compteur électronique d'inhalateur selon l'une quelconque des revendications précédentes dans lequel le premier degré sélectionné de mouvement d'actionnement linéaire ne comprime pas le ressort de rappel (108), et dans lequel le second degré sélectionné de mouvement d'actionnement linéaire comprime le ressort de rappel (108).

6. Compteur électronique d'inhalateur selon la revendication 2 dans lequel au moins une partie du module de détection de respiration d'inhalateur est conçu pour s'ajuster au sein d'au moins une partie du logement d'actionneur (402) d'inhalateur.

7. Compteur électronique d'inhalateur selon la revendication 2 ou 5 dans lequel le dispositif inhalateur est actionné en appuyant sur le compteur électronique d'inhalateur (100) par rapport au logement d'actionneur (402).

8. Compteur électronique d'inhalateur selon la revendication 6 dans lequel lorsque le dispositif inhalateur est actionné au moins une partie du compteur électronique d'inhalateur (100) s'ajuste au sein du logement d'actionneur (402) d'inhalateur.

9. Compteur électronique d'inhalateur selon l'une quelconque des revendications précédentes, comprenant en outre un affichage numérique (124), dans lequel l'affichage est configuré pour afficher au moins l'un parmi le nombre compté d'actionnements linéaires du dispositif inhalateur, et/ou le nombre d'actionnements linéaires restants du dispositif inhalateur, dans lequel le nombre d'actionnements linéaires restants du dispositif inhalateur est calculé par le nombre compté d'actionnements linéaires du dispositif inhalateur soustrait d'un nombre maximal prédéterminé d'actionnements linéaires du dispositif inhalateur.
